# EUROPEAN PATENT SPECIFICATION

(11) **EP 2 966 988 B1**
(45) Date of publication and mention of the grant of the patent: **16.08.2023**
(21) Application number: 14708916.3
(22) Date of filing: 11.03.2014
(51) Int. Cl.: A01H 5/00, A01H 5/08, C12Q 1/68

(54) **DARK STEM CUCUMBER PLANTS**
GURKENPFLANZEN MIT DUNKLEM STAMM
PLANTES DE CONCOMBRE SOUCHE SOMBRE

(30) Priority: 11.03.2013 EP 13158611; 11.03.2013 US 201313793008
(43) Date of publication of application: 20.01.2016
(73) Proprietor: Rijk Zwaan Zaadteelt en Zaadhandel B.V., 2678 KX De Lier (NL)
(72) Inventor: HAARING, Cornelis, NL-3155 GG Maasland (NL)
(74) Representative: van Someren, Petronella F. H. M.
(86) International application number: PCT/EP2014/054722
(87) International publication number: WO 2014/140026

(56) References cited:
- WO-A1-2007/042070
- WO-A1-2010/057960
- WO-A1-2011/144672
- ROB E SCHOUTEN ET AL: "Predicting keeping quality of batches of cucumber fruit based on a physiological mechanism", POSTHARVEST BIOLOGY AND TECHNOLOGY, vol. 26, no. 2, 1 September 2002 (2002-09-01), pages 209-220, XP055072482, ISSN: 0925-5214, DOI: 10.1016/S0925-5214(02)00017-0
- HOVI-PEKKANEN ET AL: "Effects of interlighting on yield and external fruit quality in year-round cultivated cucumber", SCIENTIA HORTICULTURAE, ELSEVIER SCIENCE PUBLISHERS, XX, vol. 116, no. 2, 26 December 2007 (2007-12-26), pages 152-161, XP022524546, ISSN: 0304-4238, DOI: 10.1016/J.SCIENTA.2007.11.010
- JOLIFFE P A ET AL: "PREDICTORS OF SHELF LIFE IN LONG ENGLISH CUCUMBER", JOURNAL OF THE AMERICAN SOCIETY FOR HORTICULTURAL SCIENCE, AMERICAN SOCIETY FOR HORTICULTURAL SCIENCE, ALEXANDRIA, VA, US, vol. 122, no. 5, 1 January 1997 (1997-01-01), pages 686-690, XP009066473, ISSN: 0003-1062

## Description

The present invention relates to a new cucumber plant that produces fruits that have a darker green colour as a component of extended storability. The invention also relates to phenotypic and/or molecular markers to predict the darker green colour in an early plant stage.

The increase of storability of cucumber is traditionally one of the major goals of cucumber breeding. Storability, or shelf life, consists of multiple aspects, the most important one being fruit colour. Fruit colour as an aspect of extended storability can again be subdivided into two components: the initial fruit colour, and the ability to stay green for an extended period of time. Both components are largely influenced by the genetic background of the cucumber plant, but environmental effects also play a role.

During a breeding process, initial fruit colour can only be observed phenotypically, in the adult plant stage, after fruit harvest. The development of cucumbers with an improved storability based on an improved initial fruit colour is therefore time-consuming, since the breeding cycles are long and many generations are needed to ensure the transfer of the trait. Progress in development for longer shelf-life, i.e. in an increase of days that a cucumber fruit can be stored while the colour remains acceptable for consumption, is slow.

An improved fruit colour during storage of cucumber fruits is desirable from a commercial point of view. Various measures are taken post-harvest to reduce deterioration of cucumber fruits, such as shrink-wrapping or wax-coating. From an environmentally conscious perspective shrink-wrapping is undesirable since it leads to additional waste. Both shrink-wrapping and wax-coating also require additional handling of the fruits which incurs costs and can lead to blemishes. The additional material that is needed for these measures adds up to the costs as well.

During research that led to the present invention a new cucumber plant was created, the fruits of which have a darker initial fruit colour, which darker fruit colour can be predicted phenotypically, and/or genetically through molecular markers, in an early plant stage. The darker fruit colour can serve as a component of extended storability

It is an object of the present invention to provide cucumber plants that generate fruits with an extended storability.

It is an object of the present invention to provide cucumber plants (*Cucumis sativus*) that carry a genetic determinant which leads to fruits with a darker green colour.

It is a further object of the present invention to provide a phenotypic marker to identify cucumber plants in seedling stage that carry the genetic determinant of the invention.

It is an object of the present invention to provide molecular markers to identify the genetic determinant of the invention.

The present invention thus provides a cucumber plant of the species *Cucumis sativus* carrying a genetic determinant that leads to a darker green stem in the seedling stage that is predictive of a darker green colour of the fruits as compared to an isogenic cucumber plant not carrying the said genetic determinant, wherein said genetic determinant is genetically linked with molecular markers E13/M51-154.68 and/or E16/M60-086.74, and wherein the chlorophyll content in the stem of the plant at seedling stage is at least 40 µg/g higher or at least 30% higher than the chlorophyll content in the stem of an isogenic cucumber plant at seedling stage not carrying the said genetic determinant, that is grown under the same conditions.

The present invention further relates the cucumber plant of the invention, wherein the genetic determinant is as comprised in a cucumber plant representative seed of which was deposited with the NCIMB under deposit number NCIMB 41859.

In one embodiment the genetic determinant that leads to a darker green stem in the seedling stage that is predictive of a darker green colour of the fruits as compared to an isogenic cucumber plant not carrying the said genetic determinant is introgressed from a cucumber plant comprising said genetic determinant, representative seed of which was deposited with the NCIMB under deposit number NCIMB 41859.

During research that led to the present invention new *Cucumis sativus* plants were created. The seedling stem of the new cucumber plant is chlorophyll rich, which can already be observed within one week after germination. Observation can be done visually, as the stem is very dark green, or the chlorophyll content in the stem can be measured. In a later plant stage both the leaf- and fruit stems also show a darker green colour and/or an increase in the chlorophyll content. In the fruit picking stage the fruit colour is marked as extremely dark green. In addition, after harvest the fruit has an increased conservation or storability quality. All above mentioned parts of a plant of the invention are darker and/or higher in chlorophyll content as compared to an isogenic cucumber plant grown under the same conditions, which is genetically the same but does not carry the genetic determinant of the invention.

The trait of the present invention is determined by a monogenic dominant or at least incomplete dominant genetic determinant. Therefore the genetic determinant can be present in homozygous or heterozygous state to result in the phenotypic trait of the invention. The fruits carrying the genetic determinant of the invention show a darker green colour as compared to cucumber fruits of an isogenic plant not carrying the said genetic determinant.

Herein disclosed is the said genetic determinant which is linked with any of the polymorphic molecular AFLP markers E13/M51-143.48 and/or E13/M51-154.68 and/or E16/M60-086.74 in a cucumber plant representative seed of which was deposited with the NCIMB under deposit number NCIMB 41859.

Further disclosed herein is a cucumber plant comprising the genetic determinant of the invention, wherein the genetic determinant is linked with molecular marker E13/M51-143.48.

The cucumber plant of the invention is obtainable by crossing a first cucumber plant with a second cucumber plant, wherein at least one of the said plants is grown from seed of which a representative sample was deposited with the NCIMB under deposit number NCIMB 41859, or a progeny plant thereof, optionally selfing the resulting F1, and selecting for plants that have one or more of the following characteristics:
a) they have at the seedling stage a darker green stem as compared to the stem of a seedling of an isogenic cucumber plant not carrying the said genetic determinant, which darker green stem is predictive of the production of fruits having a darker green colour at harvest stage as compared to the fruits of an isogenic cucumber plant not carrying the said genetic determinant under the same conditions, and/or
b) they have at the seedling stage an increase in the content of chlorophyll in the stem as compared to chlorophyll content in the stem of a seedling of an isogenic cucumber plant not carrying the said genetic determinant, which increase in the content of chlorophyll is predictive of the production of fruits having a darker green colour at harvest stage as compared to the fruits of an isogenic cucumber plant not carrying the said genetic determinant under the same conditions.

Either one or both of the first and second cucumber plants used to obtain a cucumber plant of the invention thus carries the said genetic determinant that results in a darker green stem in the seedling stage that is predictive of a darker green colour of the fruits.

In the deposited seeds, the genetic determinant is linked with any of the molecular markers E13/M51-143.48 and/or E13/M51-154.68 and/or E16/M60-086.74. This marker can also be linked to the genetic determinant that is comprised in either or both cucumber plants that are used as parents in a cross to transfer the dark stem phenotype to other plants, but the presence of at least one of the mentioned markers is not essential as long as the genetic determinant causing the phenotype is present. Furthermore, a plant of the invention showing the dark stem phenotype as described herein is still a plant of the invention when the genetic determinant underlying the phenotype is present therein but the markers no longer are.

It is clear that the parent that provides the genetic determinant that leads to the trait of the invention is not necessarily a plant grown directly from the deposited seeds. The parent can also be a progeny plant from the deposited seed, obtained by for example selfing or crossing, or a progeny plant from seeds that are identified to have obtained the genetic determinant that leads to the trait of the invention by other means.

Furthermore disclosed herein is the genetic determinant as defined herein, which genetic determinant is selected from a group comprising: a gene, an allele, a gene construct, a QTL, a promoter, an isolated gene, a transgene, a DNA sequence.

Plants of the invention can be identified on the basis of the following selection characteristics:
a) they produce fruits having a darker green colour at harvest stage and during storage up to 14 days, preferably up to 17 days, more preferably up to 19 days, most preferably up to 21 days after harvest, as compared to the fruits of an isogenic cucumber plant not carrying the said genetic determinant that is grown and stored under the same conditions, and/or
b) they have at the seedling stage a darker green stem as compared to the stem of a seedling of an isogenic cucumber plant not carrying the said genetic determinant, and/or
c) they have at the seedling stage an increase in the content of chlorophyll in the stem as compared to the stem of a seedling of an isogenic cucumber plant not carrying the said genetic determinant.

Also disclosed herein is that the occurrence of one or more characteristics in a selected plant is genetically linked with any of the molecular markers E13/M51-143.48 and/or E13/M51-154.68 and/or E16/M60-086.74.

In a cucumber plant of the invention the chlorophyll content in the stem of the plant at seedling stage is at least, in order of increased preference, 40 µg/g, 70 µg/g, 100 µg/g, 120 µg/g, 150 µg/g, 180 µg/g, 200 µg/g higher than the chlorophyll content in the stem of an isogenic cucumber plant at seedling stage not carrying the said genetic determinant, that is grown under the same conditions.

The chlorophyll content in the stem of a cucumber seedling carrying the genetic determinant as disclosed herein is preferably higher than 140 µg/g. The chlorophyll content in the seedling stem is suitably not higher than 1000 µg/g.

In a cucumber plant of the invention the chlorophyll content in the stem of the plant at seedling stage is at least, in order of increased preference, 30% higher, 60% higher, 90% higher, 120% higher, 150% higher, 180% higher, 200% higher, 230% higher, 270% higher, 300% higher than the chlorophyll content in the stem of an isogenic cucumber plant at seedling stage not carrying the said genetic determinant, that is grown under the same conditions.

The darker green stem of a seedling of a cucumber plant of the invention can also be scored visually. On a scale of 1-5, the seedling stem of a cucumber plant of the invention is at least 1 grade darker, preferably at least 2 grades darker, than the colour of the stem of an isogenic cucumber plant at seedling stage not carrying the genetic determinant of the invention. To compare the colours of the stem, or to compare the chlorophyll contents, it is essential that the seedlings are grown under the same conditions.

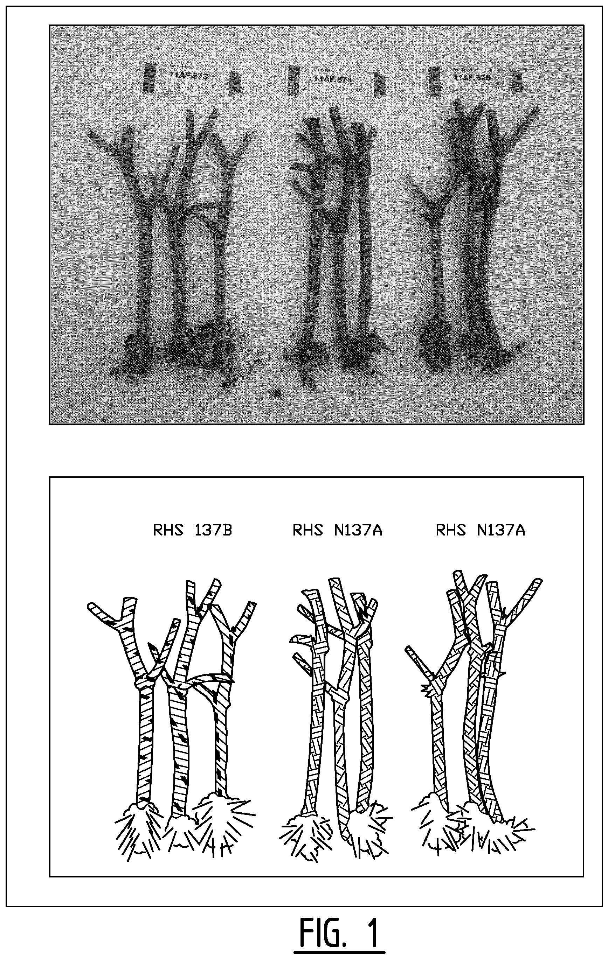

A visual comparison of the stem colour at 21 days after sowing is shown schematically in **Figure 1****.** The actual colours of the seedling stems depicted in **Figure 1** were determined to be 137B for the normal stem and N137A for the dark stem based on the colour chart of the Royal Horticultural Society. However, it is not necessary that a plant of the invention has exactly colour N137A. A plant of the invention has to have a colour that differs from the colour of an isogenic plant without the genetic determinant to the same or a similar extent as the difference between 137B and N137A. The RGB, CIELab and CIELCh values for the two colours are listed in the **Table 1** below.

**Table 1**

| Comparison of colour values | | | | | | | | | |
|---|---|---|---|---|---|---|---|---|---|
| RHS code | sRGB | | | CIE Lab D65/10° | | | CIE LCh D65/10° | | |
| | R | G | B | L | a | b | L | C | h |
| 137B | 91 | 111 | 81 | 45 | -13 | 14 | 45 | 19 | 134 |
| N137A | 81 | 91 | 69 | 37 | -8 | 11 | 37 | 14 | 127 |

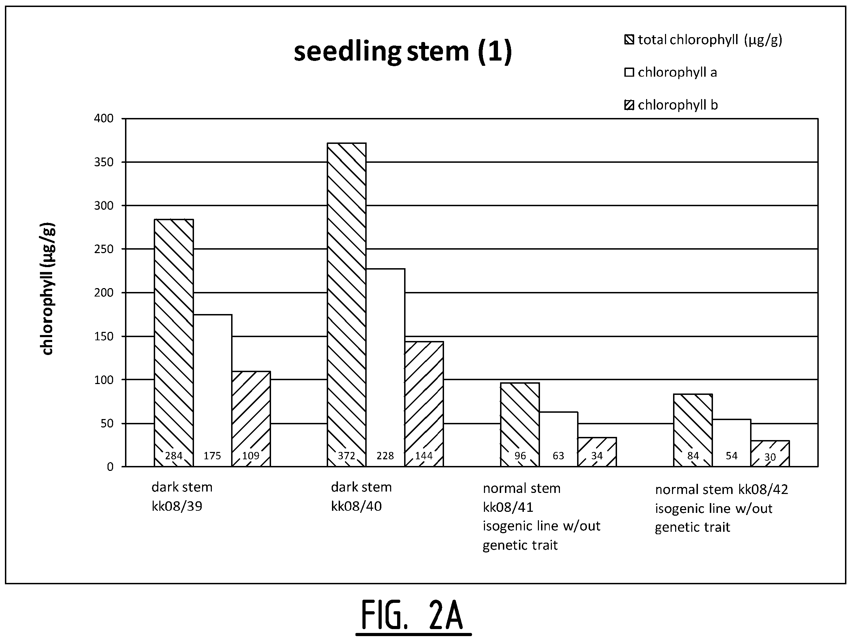

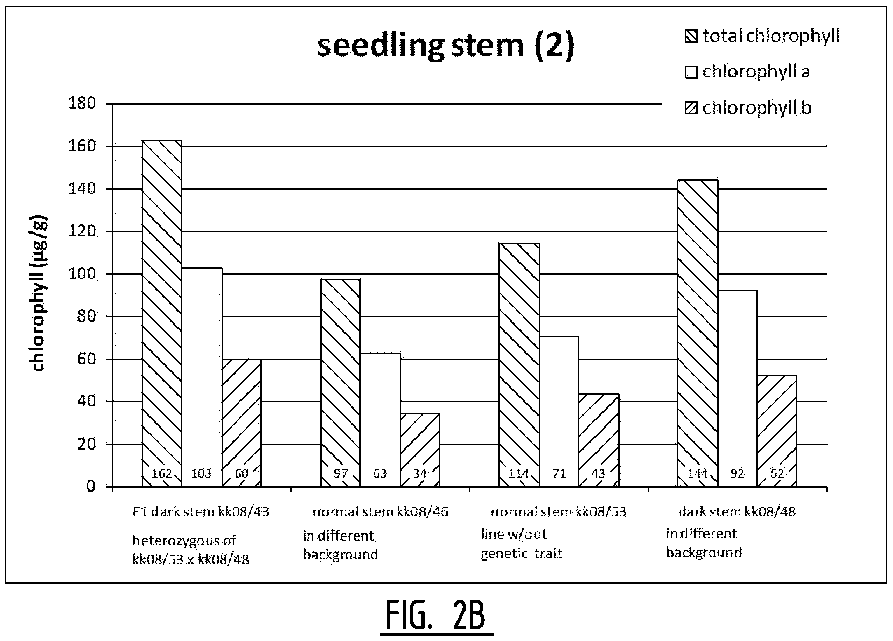

Measurements and comparison of chlorophyll content between seedlings of plants of the invention and isogenic lines without the genetic determinant can be found in **Figure 2****.**

The darker green seedling stem can optionally already be observed in a seedling that is one week old, wherein the hypocotyl, below the cotyledons, shows the darker stem. Preferably the stem colour or chlorophyll content is compared in seedlings that are 2-3 weeks old, wherein also the stem above the cotyledons shows the darker colour and/or the increase in chlorophyll content.

Throughout the growing period of the cucumber plant the stem remains darker, and also the stems of the leaves and later on of the fruits are visibly darker than from cucumber plants that do not have the genetic determinant of the invention.

Additionally disclosed herein is a cucumber plant carrying the genetic determinant of the invention that leads to a darker green stem and/or an increase in the chlorophyll content in the seedling stage, wherein the green colour of the fruits produced by said plant is at least 2 grades darker, preferably at least 3 grades darker, on a scale of 1-9, than the colour of the fruit of an isogenic cucumber plant not carrying said genetic determinant, that is grown and stored under the same conditions. The colour of the fruits is determined at harvest stage.

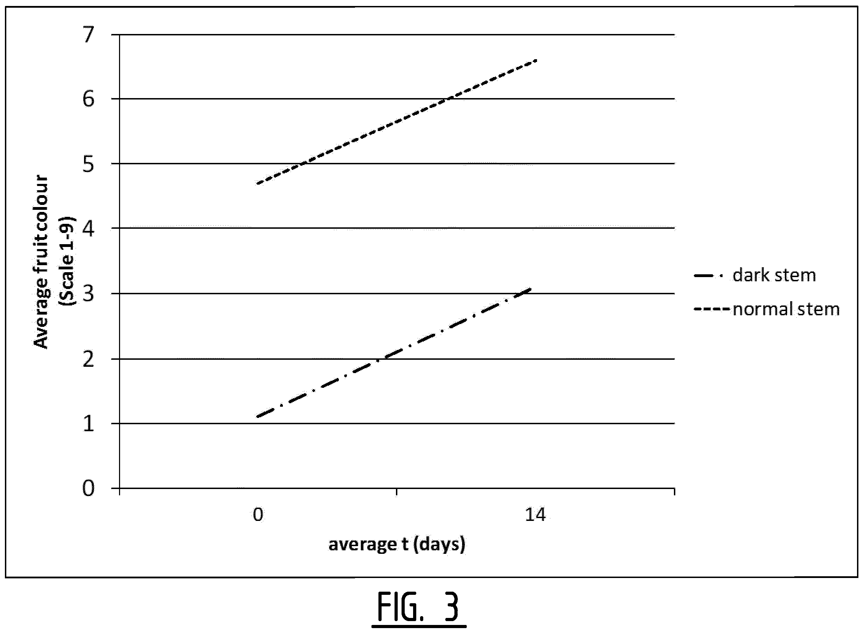

In a preferred embodiment of the invention the colour of the fruits remains at least 2 grades darker, preferably at least 3 grades darker, on a scale of 1-9, from harvest stage until at least 14 days of storage, preferably until at least 17 days of storage, more preferably until at least 19 days of storage, most preferably until at least 21 days of storage when compared to the fruit of an isogenic cucumber plant not carrying the genetic determinant, that is grown and stored under the same conditions (**Table 3,** **Figure 3**).

After harvesting the cucumber fruits at their usual harvesting stage, cucumbers are preferably stored in climate chambers. A colour scale of 1-9 can be used to visually determine the difference in colour of the fruits. As used herein, a score of 1 is the darkest green fruit colour, a score of 9 is a very light or yellow fruit colour.

The colour categories are changing gradually and can be indicated as follows: 1. extremely dark green; 2. very dark green; 3. dark green; 4. green; 5. medium green; 6. light green; 7. light green with first indication of yellowing; 8. partly yellowing; 9. yellow. A standard European greenhouse cucumber type in this scale typically has a colour around score 4 or 5. Other cucumber types may have a starting colour that scores somewhat higher (lighter) or lower (darker). A cucumber fruit of the invention preferably has a score of around 3, more preferably a score of around 2, most preferably a score between 1 and 2.

Optionally another scale can be used, for example a scale of 1-5, wherein the colour difference is at least 1 grade, preferably at least 1.5 grades. The scores 1-5 then translate to scores 1, 3, 5, 7, and 9 of the scale of 1-9 described above.

The genetic determinant that leads to a darker green stem and/or an increase in the chlorophyll content in the seedling stage preferably also leads to an increase in the chlorophyll content of the skin of the fruit. The darker green stem can be used as a marker for the trait of the darker fruits and allows early identification of plants carrying the genetic determinant leading to such darker green fruits.

In one embodiment a cucumber plant of the invention has a darker green colour of the fruit that relates to an increase in the chlorophyll content of the skin of the fruit of at least, in order of increased preference, 100 µg/g, 130 µg/g, 160 µg/g, 200 µg/g, 240 µg/g as compared to the fruit of an isogenic cucumber plant not carrying the said genetic determinant, that is grown and optionally stored under the same conditions. The comparison of chlorophyll content of the skin of the fruit is suitably done at harvest stage.

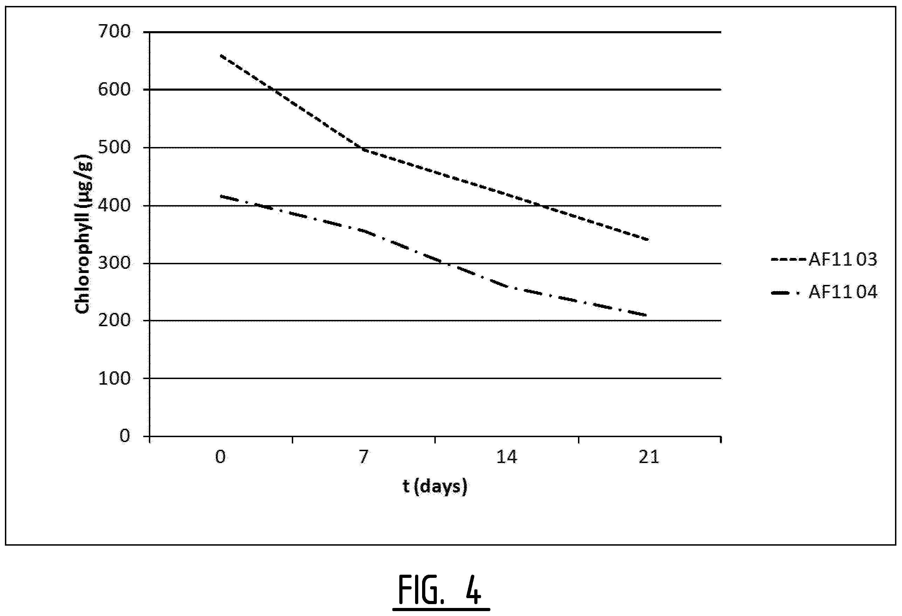

In one embodiment the invention relates to a cucumber plant carrying the genetic determinant of the invention, wherein the darker green colour of the fruit of the plant of the invention relates to an increase in the chlorophyll content of the skin of the fruit of at least, in order of increased preference, 30%, 40%, 50%, 60%, 70% as compared to the fruit of an isogenic cucumber plant not carrying the said genetic determinant, that is grown and optionally stored under the same conditions (**Table 4,** **Figure 4**). The comparison of chlorophyll content of the skin of the fruit is suitably done at harvest stage. The total chlorophyll content in the skin of a cucumber fruit of the invention is suitably not higher than 1000 µg/g.

During storage of cucumber fruits, the chlorophyll content in the skin of the fruits decreases over time. The chlorophyll content in the skin of a fruit of the invention remains significantly higher during at least 14 days of storage, preferably until at least 21 days of storage, as compared to an isogenic cucumber fruit that does not carry the genetic determinant of the invention. Storage as mentioned herein is suitably done at 17°C.

Also disclosed herein is a cell of a cucumber plant as claimed. Such cell may be either in isolated form or may be part of the complete cucumber plant or parts thereof and then still constitutes a cell of the invention because such a cell harbours in its genetic constitution the genetic information that leads to the characteristics that define the cucumber plant of the invention. Each cell of cucumber plants of the invention carries the genetic information, i.e. the genetic determinant, that leads to phenotypic expression of said trait. Such a cell of the invention may also be a regenerable cell that can be used to regenerate a new cucumber plant of the invention. In the context of this application "of the invention" means carrying a genetic determinant leading to the expression of a darker green stem in the seedling stage and a darker green fruit colour. The darker green colour contributes to a better storability.

Further disclosed herein is tissue of a plant as claimed. The tissue can be undifferentiated tissue or already differentiated tissue. Undifferentiated tissues are for example stem tips, anthers, petals, pollen and can be used in micropropagation to obtain new plantlets that are grown into new plants of the invention. The tissue can also be grown from a cell of the invention.

The invention according to a further aspect thereof relates to seeds of a plant as claimed. Although the seeds do not show the genetic trait of the cucumber plant of the invention, they harbour the genetic information, i.e. the genetic determinant, that when a plant is grown from the seeds makes this plant a plant of the invention.

The invention also relates to progeny of the plants, cells, tissues and seeds of the invention. Such progeny can in itself be plants, cells, tissues or seeds.
As used herein the word "progeny" is intended to mean the first and all further descendants from a cross with a plant of the invention that comprises the genetic determinant that leads to a darker green stem in the seedling stage that is predictive of a darker green colour of the fruits as compared to an isogenic cucumber plant not carrying the said genetic determinant. Progeny of the invention are descendants of any cross with a plant of the invention that carries the genetic determinant that leads to a darker green stem in the seedling stage that is predictive of a darker green colour of the fruits.

"Progeny" also encompasses plants that carry the genetic determinant that leads to a darker green stem in the seedling stage that is predictive of a darker green colour of the fruits as compared to an isogenic cucumber plant not carrying the said genetic determinant and are obtained from other plants or progeny of plants of the invention by vegetative propagation or multiplication.

The invention thus further relates to seed of the claimed plant and to parts of the plant that are suitable for sexual reproduction. Such parts are for example selected from the group consisting of microspores, pollen, ovaries, ovules, embryo sacs and egg cells. In addition, the invention relates to parts of the plant that are suitable for vegetative reproduction, in particular cuttings, roots, stems, cells, and protoplasts. The parts of the plants as mentioned above are considered propagation material.

According to a further aspect thereof the invention concerns a tissue culture of the claimed plant which is also propagation material. The tissue culture comprises regenerable cells. Such tissue culture can be selected or derived from any part of the plant, in particular from leaves, pollen, embryos, cotyledons, hypocotyls, meristematic cells, roots, root tips, anthers, flowers, seeds and stems. The tissue culture can be regenerated into a plant carrying the genetic determinant of the invention. Suitably a regenerated plant expresses the phenotype of a darker green stem in the seedling stage that is predictive of a darker green colour of the fruits as compared to an isogenic cucumber plant not carrying the said genetic determinant.

An isogenic cucumber plant of the cucumber plant of the invention can be obtained by one or more backcrosses with the parent that has received the genetic determinant of the invention. After a number of backcrosses the genetic constitution of the resulting plant is essentially the same as the genetic constitution of the starting plant except for the trait of the invention that is introgressed. The plant of the invention and the starting plant are then isogenic. Comparisons of colour are made between isogenic plants to avoid any potential influence of other genes on the colour.

The present invention is in particular useful for so-called long cucumber types and European greenhouse cucumber types.

Further disclosed herein is a hybrid seed and to a method of producing hybrid seed comprising crossing a first parent plant with a second parent plant and harvesting the resultant hybrid seed, wherein said first parent plant and/or said second parent plant has the genetic determinant of the invention. When the parent has the genetic determinant of the invention in homozygous form, the resulting hybrid plant is also a plant as claimed.

Also disclosed herein are cucumber plants of the invention that carry the genetic determinant of the invention which leads to a darker green stem in the seedling stage that is predictive of a darker green colour of the fruits as compared to an isogenic cucumber plant not carrying the said genetic determinant, and that have acquired said determinant by introduction of the genetic information that is responsible for the trait from a suitable source, either by conventional breeding, or genetic modification, in particular by cisgenesis or transgenesis. Cisgenesis is genetic modification of plants with a natural gene, coding for an (agricultural) trait, from the crop plant itself or from a sexually compatible donor plant. Transgenesis is genetic modification of a plant with a gene from a non-crossable species or a synthetic gene.

Furthermore disclosed herein is the source from which the genetic determinant of the invention is acquired is formed by plants grown from seeds of which a representative sample was deposited under accession number NCIMB 41859, or from the deposited seeds NCIMB 41859, or from sexual or vegetative descendants thereof, or from another source comprising the genetic determinant that leads to the trait of the invention, or from a combination of these sources.

Additionally disclosed herein are non-transgenic *Cucumis sativus* plants. The source for acquiring the genetic determinant of the invention, to obtain a plant of the invention that has a darker green stem which is predictive of a darker green colour of the fruits, is suitably a *Cucumis sativus* plant that carries the genetic determinant as comprised in NCIMB 41859, or alternatively a plant of a *Cucumis* species that carries said gene and that can be crossed with *Cucumis sativus.* Optionally, after crossing with a related species, techniques such as embryo rescue, backcrossing, or other techniques known to the skilled person can be performed to obtain seeds of the interspecific cross, which seeds can be used as the source for further development of a non-transgenic *Cucumis sativus* plant that shows a darker green stem in the seedling stage that is predictive of a darker green colour of the fruits as compared to an isogenic cucumber plant not carrying the said genetic determinant.

Also disclosed herein is the germplasm of plants of the invention. The germplasm is constituted by all inherited characteristics of an organism and according to the invention encompasses at least the trait of the invention. The germplasm can be used in a breeding programme for the development of cucumber plants having a darker green stem that is predictive of a darker green colour of the fruits as compared to an isogenic cucumber plant not carrying the said genetic determinant. The use of the germplasm that comprises the genetic determinant leading to a darker green stem and darker green fruits in breeding is also disclosed herein.

Further disclosed herein is the use of the genetic determinant leading to the trait of the invention, which genetic determinant is genetically linked with any of the molecular markers E13/M51-143.48 and/or E13/M51-154.68 and/or E16/M60-086.74, for the development of cucumber plants that have a darker green stem in the seedling stage that is predictive of a darker green colour of the fruits at harvest stage as compared to an isogenic cucumber plant not carrying the said genetic determinant

Also disclosed herein is a cucumber fruit that is produced by a plant of the inventionand a food product, comprising the fruit of a cucumber plant as claimed, or parts thereof. The food product may be in processed form.

Further disclosed herein is a method for production of a cucumber plant comprising the genetic determinant that leads to a darker green stem in the seedling stage that is predictive of a darker green colour of the fruits as compared to an isogenic cucumber plant not carrying the said genetic determinant, comprising:
a) crossing a plant comprising the genetic determinant of the invention, representative seed of which plant was deposited as NCIMB 41859, with a plant not comprising the genetic determinant to obtain an F1 population;
b) optionally performing one or more rounds of selfing and/or crossing a plant from the F1 to obtain a further generation population;
c) selecting a plant that comprises the genetic determinant that results in a darker green stem in the seedling stage that is predictive of a darker green colour of the fruits as compared to an isogenic cucumber plant not carrying the said genetic determinant, suitably by using molecular markers linked to the genetic determinant. The plant can also be selected by visual inspection of the stem of the seedling for a darker green colour than the parent plant that does not comprise the genetic determinant of the invention.

Furthermore disclosed herein is a method of introducing another desired trait into a cucumber plant comprising the trait of the invention, comprising:
a) crossing a cucumber plant comprising the genetic determinant that leads to a darker green stem in the seedling stage that is predictive of a darker green colour of the fruits as compared to an isogenic cucumber plant not carrying the said genetic determinant, representative seed of which was deposited with the NCIMB under deposit number NCIMB 41859, with a second cucumber plant that comprises the other desired trait to produce F1 progeny;
b) selecting an F1 progeny that comprises genetic determinants for the trait of the invention and for the desired trait;
c) crossing the selected F1 progeny with either parent, to produce backcross progeny;
d) selecting backcross progeny comprising genetic determinants for the desired trait and for the trait of the invention; and
e) optionally repeating steps c) and d) one or more times in succession to produce selected fourth or higher backcross progeny that comprises the desired trait and the trait of the invention. The invention includes a cucumber plant produced by this method and the cucumber fruit obtained therefrom.

Optionally selfing steps are performed after any of the crossing or backcrossing steps. Selection for a plant comprising the genetic determinant of the invention and the desired trait can alternatively be done following any crossing or selfing step of the method.

The plant of the invention comprising the genetic determinant either homozygously or heterozygously may be a plant of an inbred line, a hybrid, a doubled haploid, or of a segregating population.

Furthermore, disclosed herein is a method for the production of a cucumber plant comprising a darker green stem as defined herein by using a doubled haploid generation technique to generate a doubled haploid line that homozygously comprises the genetic determinant that leads to the darker green stem, which doubled haploid line can be crossed with a line that lacks the said genetic determinant to generate a plant of the invention that comprises the genetic determinant heterozygously.

Further disclosed herein is a method for the production of a cucumber plant comprising the genetic determinant that leads to the darker green stem of the invention, by using a seed that comprises the genetic determinant in its genome that leads to the darker green stem of the invention for growing the said cucumber plant. The seeds are suitably seeds of which a representative sample was deposited with the NCIMB under deposit number NCIMB 41859.

Also disclosed herein is a method for seed production comprising growing cucumber plants comprising the genetic determinant of the invention, which results in the phenotypic trait of the invention, allowing the plants to produce seeds, and harvesting those seeds. Production of the seeds is suitably done by crossing or selfing.

In one embodiment, the invention relates to a method for the production of a cucumber plant comprising a darker green stem that is predictive of a darker green fruit colour by using propagation material suitable for vegetative reproduction or suitable for tissue cultures of regenerable cells as defined herein.

Additionally disclosed herein is a method for the production of a cucumber plant comprising the darker green stem by using a method for genetic modification to introduce the genetic determinant of the invention that leads to a darker green stem in the seedling stage that is predictive of a darker green colour of the fruits as compared to an isogenic cucumber plant not carrying the said genetic determinant into the cucumber plant.

Further disclosed herein is a cucumber plant showing a darker green stem in the seedling stage that is predictive of a darker green colour of the fruits as compared to an isogenic cucumber plant not carrying the said genetic determinant, which plant is obtainable by any of the methods herein described.

Also disclosed herein is a method for the production of cucumber fruits, comprising growing cucumber plants with a darker green stem as described herein and allowing them to produce cucumber fruits and optionally harvesting the fruits.

The term 'genetic determinant' and 'genetic determinants' as used herein encompasses one or more QTLs, genes, or alleles. These terms are used interchangeably.

A genetic determinant can be identified by the use of a molecular marker. A genetic determinant can alternatively be identified by the position on a genetic map, or by indication of the location on a linkage group or chromosome. When a genetic determinant is not linked to a specific molecular marker any longer, but its position on a chromosome as defined on a genetic map is unaltered, this genetic determinant is still the same as when it was linked to the molecular marker. The genetic trait that it confers is therefore also still the same.

The 'genetic trait' is the trait or characteristic that is conferred by the genetic determinant. The genetic trait can be identified phenotypically, for example by performing a bioassay. However, also plant stages for which no phenotypic assay can be performed do carry the genetic information that leads to the genetic trait. 'Trait' or 'phenotypic trait' can be used instead of 'genetic trait'.

In the absence of molecular markers, or in the instance that recombination between the genetic determinant and the marker has taken place so that the marker is not predictive anymore, equivalence of genetic determinants can be determined by an allelism test. To perform an allelism test, a tester plant which is homozygous for the known determinant of the invention is crossed with material to be tested that is also homozygous for its genetic determinant. When no segregation for the trait to be observed is present in the F2 of the cross, the genetic determinants have been proven to be equivalent or the same.

When more than one gene is responsible for a certain trait, and an allelism test is done to determine equivalence, the skilled person doing the test has to make sure that all relevant genes are present homozygously for the test to work properly.

### DEPOSIT

Seeds of *Cucumis sativus* EX5004 that comprise the genetic determinant and phenotypic trait of the invention were deposited with NCIMB Ltd, Ferguson Building, Craibstone Estate, Bucksburn, Aberdeen AB21 9YA, UK on 31/08/2011 under deposit accession number NCIMB 41859.

The deposited seeds do not meet the DUS criteria which are required for obtaining plant variety protection, and can therefore not be considered to be plant varieties.

The invention will be further illustrated in the Examples that follow.

### MARKER INFORMATION

The primers used for the AFLP markers are standardized and the sequences to combine can be found at for example the following website: ttp://wheat.pw.usda.gov/ggpages/keygeneAFLPs.html.

### FIGURES

**Figure 1****:** comparison picture of cucumber dark stem and normal stem . Plant 11AF.873 has a normal stem; plant 11AF.874 has a dark stem and plant 11AF.875 is an F1 from the cross (dark stem x normal stem).
**Figure 2****:** chlorophyll measurements of cucumber seedling stems
**Figure 3****:** average cucumber fruit colour at harvest and after storage
**Figure 4****:** chlorophyll content in skins of the cucumber fruit at harvest and after storage

### EXAMPLES

### EXAMPLE 1

### Phenotypic characterization of the invention

To obtain cucumber fruits with an increased storability, one aspect forms the improvement of fruit colour, wherein a darker green fruit colour is favored. This is however a complex goal to attain in breeding.

In the germplasm of the cucumber breeding programme, a plant was developed that already several days after germination showed an unusually dark seedling stem. This phenotypic trait was monitored during the subsequent development of the plant. It appeared that succeeding plant stems, including the main stem, but also the stems, or petioles, of the leaves and later on of the fruits, displayed an unusually dark colour.

It was decided to explore whether there existed a potential benefit of the darker green stems, which was expected to be connected to an increase in the chlorophyll content, in relation to the colour of the fruits. Surprisingly it was found that the plants that showed a darker green stem colour also had a darker green fruit colour. No relation between fruit and stem colour in cucumber has ever been established earlier.

The plant comprising the darker green stem colour was crossed with a plant with a normal stem colour to observe the inheritance. It was clear that the F1 also showed a darker green stem colour, which confirms a dominant inheritance of the phenotype of this trait (Fig. 1). The fruits of said plants were compared as well, and the inheritance of the darker green fruit colour was also determined to be dominant.

After selfing the F1 plants, segregation data from F2 and further generations corresponded to the presence of a monogenetic determinant for this trait.

### EXAMPLE 2

### Biochemical characterization of the invention

The stems of the seedlings that were developed in **Example 1** were analysed for chlorophyll content. To obtain reliable comparisons, different plants containing the trait homozygously were measured, and comparison was done with isogenic lines which are genetically the same but lack the genetic determinant of the invention.

The analysis confirmed that the darker green seedling stems were a result of an increase in chlorophyll content. Both chlorophyll components that were present, chlorophyll a and chlorophyll b, were increased, resulting in a significant increase in total chlorophyll content (**Fig**. **2**).

The chlorophyll content of the seedling stem of the F1 was measured as well. This showed that in the seedling stem the biochemical feature also inherited as a completely dominant trait. The increase in chlorophyll content compared to a normal stem colour was clearly present, and was even slightly higher than the content of the parent in which the genetic determinant was homozygously present.

Again, also the observation was done on fruits of plants with an increased chlorophyll content in the stem. The measurements of the darker green fruits of these plants validated that the darker colour was once more the result of an increase in chlorophyll content in the skin of the fruits, and that this feature inherited dominantly. A clear correlation was therefore established between an increase in chlorophyll contents of stems and fruits of plants of the invention.

### EXAMPLE 3

### Molecular characterization of the invention

A population of 90 DH plants was developed from an F1 between dark stem and normal stem. 48 DH individuals were eventually used to identify AFLP markers linked to the trait, using a Bulk Segregant Analysis (BSA) approach. Half of the individuals had the dark green stem phenotype, and the other half were normal green. Both parents of the population were included in the analysis.

Two bulks of 10 individuals were initially used for screening with 192 primer combinations (PC's). From this screen, six PC's were identified to be verified on the 48 DH individuals, which resulted in 7 co-dominantly scored AFLP markers. The generated AFLP markers were further analysed for linkage to the trait of the invention. Finally, 3 AFLP markers were determined to be completely associated with the dark stem trait in this population, which markers were E13/M51-143.48, E13/M51-154.68, and E16/M60-086.74

The identified molecular markers can be used to identify the presence of the genetic determinant of the invention in plants grown from seeds as deposited under NCIMB number 41859. Other plants that comprise the same genetic determinant of the invention might also be linked to the said markers, but can optionally also be linked to any other molecular marker that is polymorphic in a certain population.

The cucumber plant of the invention was further analyzed for the presence of known shelf life QTLs, as defined in WO2007/042070. Presence of QTL1 of said application was assayed by determining whether sequence 9, as indicated in WO2007/042070 to be QTL1, was present in a cucumber plant of the present invention, such as the deposit. In addition, the presence of QTL2 was assayed by determining the presence of the marker defined in WO2007/042070 as a marker of about 137 base pairs consisting from 5' to 3' of a first primer having sequence 5, a cucumber genomic fragment, and a second primer having sequence 6. For the second marker through which said QTL2 could be identified, no polymorphisms were found in a very large set of cucumber plants.

Since WO2007/042070 does not provide a deposit for reference, a prior art cucumber variety that comprised sequence 9 as well as QTL2 of said application, was identified by marker analysis performed at KeyGene (Wageningen, Netherlands). The stem colour of this particular plant was compared with a cucumber plant of the present invention, in particular with the deposit. Results are presented in **Table 2.**

**Table 2**

| *Cucumis sativus* | QTL 1 - sequence 9 | QTL2 marker | Stem colour |
|---|---|---|---|
| Beluga F1 | H | A | Green-medium: 4-5 |
| Azabache F1 | A | A | Green-medium: 4-5 |
| NCIMB 41859 | B | B | Very dark: 2 |

| | | | |
|---|---|---|---|
| A means the marker or sequence is present homozygously H means the marker or sequence is present heterozygously B means the marker or sequence is absent | | | |

### EXAMPLE 4

### Fruit colour and chlorophyll during storage

Fruits of plants of the invention were observed for their performance during storage. The fruits were compared with fruits from isogenic plants that were genetically the same but did not comprise the genetic determinant of the invention.

Initial fruit colour was scored for fruits from both types, using a score of 1-9, in which a score of 1 was the darkest green to be observed, and 9 was a yellowed cucumber fruit. The fruits were subsequently stored at 17°C for 10 up to 19 days and graded again at the end. Fruits of the invention were at least 3 grades darker at the harvesting date, on average even 3.6 grades darker, and remained at least 2.5 grades darker after storage, on average even 3.5 grades darker (**Table 3**).

**Table 3**

| Fruit colour during storage | | | | | | | |
|---|---|---|---|---|---|---|---|
| | **days** | **start** | **end** | **colour decrease** | **start** | **end** | **colour decrease** |
| | | **fruit colour dark stem** | | | **fruit colour normal stem** | | |
| | 17 | 1.3 | 2.5 | 1.2 | 4.2 | 6.4 | 2.2 |
| | 15 | 1.1 | 2.3 | 1.2 | 4.7 | 6.1 | 1.4 |
| | 10 | 1.2 | 2 | 0.8 | 4.9 | 5.6 | 0.7 |
| | 19 | 1 | 5.2 | 4.2 | 4.5 | 7.9 | 3.4 |
| | 14 | 1 | 3.5 | 2.5 | 4.8 | 7 | 2.2 |
| | 12 | 1 | 2.8 | 1.8 | 4.8 | 6.8 | 2 |
| **Average** | **14** | **1.1** | **3.1** | **2** | **4.7** | **6.6** | **2** |

| | | | | | | | |
|---|---|---|---|---|---|---|---|
| 1 = darkest green 9 = lightest/yellow | | | | | | | |

As can be seen from the data, cucumber fruits of the invention at the end of storage still possessed a colour that was darker, which is perceived to be better, than just harvested cucumber fruits that did not comprise the genetic determinant of the invention. Therefore the darker green stem of plants of the invention is surprisingly but clearly correlated with an improvement in initial fruit colour, and in fruit colour during storage, of cucumber fruits.

In addition, chlorophyll content in the skins of the fruits was measured after harvest, after 7 days, 14 days, and 21 days. The measurements clearly confirmed that the chlorophyll content of dark green fruits of the invention started out significantly higher when compared to an isogenic line lacking the genetic determinant of the invention, and remained significantly higher during storage up to 21 days (**Table 4,** **Fig. 4**).

**Table 4**

| Chlorophyll content in fruit skin | | | | |
|---|---|---|---|---|
| line dark stem | t (days) | chlorophyll (µg/g) in fruit skin | line normal stem | chlorophyll (µg/g) in fruit skin |
| | | | | |
| AF11 03 | 0 | 658 | AF11 04 | 415 |
| AF11 03 | 7 | 496 | AF11 04 | 355 |
| AF11 03 | 14 | 418 | AF11 04 | 259 |
| AF11 03 | 21 | 340 | AF11 04 | 209 |

The chlorophyll measurements of the skins of the fruits confirmed the correlation between dark green stems and/or stems higher in chlorophyll content on the one hand, and dark green fruit colour and/or higher chlorophyll content in the skins of the fruits at harvest and during storage up till 21 days on the other hand.

### EXAMPLE 5

### Transfer of the trait of the invention

In an F2 population as was for instance developed in **Example 1,** plants comprising darker green stem could be selected phenotypically, but to immediately obtain plants that contained the trait homozygously, markers as described in **Example 3** were used. The trait of the invention appeared to be easily transferable into other backgrounds, as can also be seen in **Figure 2****.** The presence of the genetic determinant was confirmed to result in a darker green seedling stem and darker green fruits as compared to the parent that was used in the cross and did not yet contain the genetic determinant of the invention.

The genetic determinant and phenotypic trait of the invention have therefore shown to be usable in various backgrounds to improve the initial fruit colour of cucumbers, and at the same time improve the quality of cucumber fruits during a storage time of at least 10, up to at least 21 days.

## Claims

1. Cucumber plant of the species *Cucumis sativus* carrying a genetic determinant that leads to a darker green stem in the seedling stage that is predictive of a darker green colour of the fruits as compared to an isogenic cucumber plant not carrying the said genetic determinant, wherein said genetic determinant is genetically linked with molecular markers E13/M51-154.68 and/or E16/M60-086.74, and wherein the chlorophyll content in the stem of the plant at seedling stage is at least 40 µg/g higher or at least 30% higher than the chlorophyll content in the stem of an isogenic cucumber plant at seedling stage not carrying the said genetic determinant, that is grown under the same conditions, wherein the genetic determinant is as comprised in a cucumber plant of which representative seed has been deposited with the NCIMB under deposit number NCIMB 41859.

2. Cucumber plant as claimed in claim 1, wherein the genetic determinant is introgressed from a cucumber plant of which representative seed has been deposited with the NCIMB under deposit number NCIMB 41859.

3. Cucumber plant as claimed in claim 1 or 2, obtainable by crossing a first cucumber plant with a second cucumber plant, wherein at least one of the said plants is grown from seed of which a representative sample was deposited under deposit number NCIMB 41859, or a progeny plant thereof, optionally selfing the resulting F1, and selecting for plants that have one or more of the following characteristics:
a) they have at the seedling stage a darker green stem as compared to the stem of a seedling of an isogenic cucumber plant not carrying the said genetic determinant, which darker green stem is predictive of the production of fruits having a darker green colour at harvest stage as compared to the fruits of an isogenic cucumber plant not carrying the said genetic determinant under the same conditions, and/or
b) they have at the seedling stage an increase in the content of chlorophyll in the stem as compared to the stem of a seedling of an isogenic cucumber plant not carrying the said genetic determinant, which increase in the content of chlorophyll is predictive of the production of fruits having a darker green colour at harvest stage as compared to the fruits of an isogenic cucumber plant not carrying the said genetic determinant under the same conditions.

4. Cucumber plant as claimed in any one of the claims 1-3, wherein the chlorophyll content in the stem of the plant at seedling stage is at least, in order of increased preference, 40 µg/g, 70 µg/g, 100 µg/g, 120 µg/g, 150 µg/g, 180 µg/g, 200 µg/g higher than the chlorophyll content in the stem of an isogenic cucumber plant at seedling stage not carrying the said genetic determinant, that is grown under the same conditions.

5. Cucumber plant as claimed in any one of the claims 1-3, wherein the chlorophyll content in the stem of the plant at seedling stage is at least, in order of increased preference, 30% higher, 60% higher, 90% higher, 120% higher, 150% higher, 180% higher, 200% higher, 230% higher, 270% higher, 300% higher than the chlorophyll content in the stem of an isogenic cucumber plant at seedling stage not carrying the said genetic determinant, that is grown under the same conditions.

6. Cucumber plant as claimed in any of the claims 1-5, wherein the green colour of the fruit from harvest stage until at least 14 days after storage, preferably until 21 days after storage, is at least 2 grades darker, preferably at least 3 grades darker, on a scale of 1-9, than the colour of the fruit of an isogenic cucumber plant not carrying the said genetic determinant, that is grown and stored under the same conditions.

7. Cucumber plant as claimed in any of the claims 1-6, wherein the darker green colour of the fruit relates to an increase in the chlorophyll content of the skin of the fruit of at least, in order of increased preference, 100 µg/g, 130 µg/g, 160 µg/g, 200 µg/g, 240 µg/g as compared to the fruit of an isogenic cucumber plant not carrying the said genetic determinant, that is grown under the same conditions.

8. Cucumber plant as claimed in any of the claims 1-7, wherein the darker green colour of the fruit at harvest stage relates to an increase in the chlorophyll content of the skin of the fruit of at least, in order of increased preference, 30%, 40%, 50%, 60%, 70% as compared to the fruit of an isogenic cucumber plant not carrying the said genetic determinant, that is grown under the same conditions.

9. Cucumber seed, wherein the plant that can be grown from the seed comprises the genetic determinant as defined in any of the claims 1-8.

10. Progeny of a cucumber plant as claimed in any one of the claims 1-8 or of cucumber seed as claimed in claim 9, comprising the genetic determinant as defined in any of the claims 1-8.

11. Propagation material suitable for producing a plant as claimed in any one of the claims 1-8, wherein the propagation material is suitable for sexual reproduction, and is in particular selected from microspores, pollen, ovaries, ovules, embryo sacs and egg cells, or is suitable for vegetative reproduction, and is in particular selected from cuttings, roots, stems, cells, protoplasts, or is suitable for tissue cultures of regenerable cells, and is in particular selected from leaves, pollen, embryos, cotyledons, hypocotyls, meristematic cells, roots, root tips, anthers, flowers, seeds and stems, wherein the plant produced from the propagation material comprises the genetic determinant as defined in any of the claims 1-8.

12. Use of a molecular marker defined as E13/M51-154.68, or E16/M60-086.74 for the identification of a cucumber plant that has a darker green stem in the seedling stage that is predictive of a darker green colour of the fruits at harvest stage as compared to an isogenic cucumber plant not carrying the said genetic determinant.

13. Molecular marker for the identification of the genetic determinant as defined in any of the claims 1-7, which molecular marker is defined as E13/M51-154.68, or E16/M60-086.74.

14. A method for the production of a cucumber plant comprising a darker green stem as defined in any one of the claims 1-8, by using a doubled haploid generation technique to generate a doubled haploid line that homozygously comprises the genetic determinant as defined in any one of the claims 1-8 that leads to the darker green stem.

15. A method for the production of a cucumber plant comprising a darker green stem that is predictive of a darker green fruit colour, by using propagation material suitable for vegetative reproduction or suitable for tissue cultures of regenerable cells as defined in claim 11.

16. A method for the production of a cucumber plant comprising the genetic determinant as defined in any one of the claims 1-8, by growing a cucumber plant from a seed that comprises the genetic determinant as defined in any one of the claims 1-8 in its genome.

17. The method as claimed in claim 16, wherein the seed is seed deposited with the NCIMB under deposit number NCIMB 41859.

## Patentansprüche

1. Gurkenpflanze der Spezies Cucumis sativus, die eine genetische Determinante trägt, welche im Keimlingsstadium zu einem dunkleren grünen Stängel führt, der im Vergleich zu einer isogenen, die genetische Determinante nicht tragenden Gurkenpflanze auf eine dunklere grüne Farbe der Früchte schließen lässt, wobei die genetische Determinante mit den Molekularmarkern E13/M51-154.68 und/oder E16/M60-086.74 genetisch verknüpft ist, und wobei der Chlorophyllgehalt im Stängel der Pflanze im Keimlingsstadium um mindestens 40 µg/g höher oder mindestens 30 % höher ist als der Chlorophyllgehalt im Stängel einer isogenen Gurkenpflanze im Keimlingsstadium, welche die genetische Determinante nicht trägt und welche unter den gleichen Bedingungen gewachsen ist, wobei die genetische Determinante in einer Gurkenpflanze enthalten ist, deren repräsentatives Saatgut bei der NCIMB unter der Hinterlegungsnummer NCIMB 41859 hinterlegt wurde.

2. Gurkenpflanze nach Anspruch 1, wobei die genetische Determinante von einer Gurkenpflanze introgressiv eingeführt wird, deren repräsentativer Samen bei der NCIMB unter der Hinterlegungsnummer NCIMB 41859 hinterlegt wurde.

3. Gurkenpflanze nach Anspruch 1 oder 2, erhältlich durch Kreuzen einer ersten Gurkenpflanze mit einer zweiten Gurkenpflanze, wobei zumindest eine der Pflanzen aus Saatgut gezogen wird, von dem eine repräsentative Probe unter der Hinterlegungsnummer NCIMB 41859 hinterlegt wurde, oder aus einer Nachkommenschaft davon, gegebenenfalls durch Selbstbefruchtung der resultierenden F1, sowie durch Selektion von Pflanzen, die eines oder mehrere der folgenden Merkmale aufweisen:
a) im Vergleich zum Stängel eines Keimlings einer isogenen, die genetische Determinante nicht tragenden Gurkenpflanze, einen dunkleren grünen Stängel im Keimlingsstadium, wobei der dunklere grüne Stängel, im Vergleich zu den Früchten einer isogenen, die genetische Determinante nicht tragenden Gurkenpflanze, unter denselben Bedingungen, auf die Bildung von Früchten mit einer dunkleren grünen Farbe im Erntestadium schließen lässt, und/oder
b) im Vergleich zum Stängel eines Keimlings einer isogenen, die genetische Determinante nicht tragenden Gurkenpflanze, eine Zunahme des Chlorophyllgehalts im Stängel im Keimlingsstadium, wobei die Zunahme des Chlorophyllgehalts im Vergleich zu den Früchten einer isogenen, die genetische Determinante nicht tragende Gurkenpflanze, unter denselben Bedingungen, auf die Bildung von Früchten mit einer dunkleren grünen Farbe im Erntestadium schließen lässt.

4. Gurkenpflanze nach einem der Ansprüche 1 bis 3, wobei der Chlorophyllgehalt im Stängel der Pflanze im Keimlingsstadium mindestens, in der Reihenfolge erhöhter Präferenz, 40 µg /g, 70 µg /g, 100 µg /g, 120 µg /g, 150 µg /g, 180 µg /g, 200 µg /g höher ist als der Chlorophyllgehalt im Stängel einer isogenen Gurkenpflanze im Keimlingsstadium, welche die genannte genetische Determinante nicht trägt und welche unter den gleichen Bedingungen gewachsen ist.

5. Gurkenpflanze nach einem der Ansprüche 1 bis 3, wobei der Chlorophyllgehalt im Stängel der Pflanze im Keimlingsstadium mindestens, in der Reihenfolge erhöhter Präferenz, 30% höher, 60% höher, 90% höher, 120% höher, 150% höher, 180% höher, 200% höher, 230% höher, 270% höher, 300% höher ist als der Chlorophyllgehalt im Stängel einer isogenen Gurkenpflanze im Keimlingsstadium, welche die genetische Determinante nicht trägt und welche unter den gleichen Bedingungen gewachsen ist.

6. Gurkenpflanze nach einem der Ansprüche 1 bis 5, wobei die grüne Farbe der Frucht vom Erntestadium bis mindestens 14 Tage nach der Lagerung, vorzugsweise bis 21 Tage nach der Lagerung, auf einer Skala von 1 bis 9 mindestens 2 Stufen dunkler, vorzugsweise mindestens 3 Stufen dunkler ist als die Farbe der Frucht einer isogenen Gurkenpflanze, welche die genetische Determinante nicht trägt und welche unter den gleichen Bedingungen gezogen und gelagert wurde.

7. Gurkenpflanze nach einem der Ansprüche 1 bis 6, **dadurch gekennzeichnet, dass** die dunklere grüne Farbe der Frucht auf eine Zunahme des Chlorophyllgehalts der Fruchthaut von mindestens, in der Reihenfolge erhöhter Präferenz, 100 µg /g, 130 µg /g, 160 µg /g, 200 µg /g, 240 µg /g im Vergleich zu den Früchten einer isogenen Gurkenpflanze, welche die genetische Determinante nicht trägt und welche unter den gleichen Bedingungen gewachsen ist, zurückzuführen ist.

8. Gurkenpflanze nach einem der Ansprüche 1 bis 7, **dadurch gekennzeichnet, dass** die dunklere grüne Farbe der Frucht im Erntestadium auf eine Zunahme des Chlorophyllgehalts der Fruchthaut von mindestens, in der Reihenfolge erhöhter Präferenz, 30 %, 40 %, 50 %, 60 %, 70 % im Vergleich zu der Frucht einer isogenen Gurkenpflanze, welche die genetische Determinante nicht trägt und welche unter den gleichen Bedingungen gewachsen ist, zurückzuführen ist.

9. Gurkensamen, wobei die Pflanze, die aus diesem Samen gezogen werden kann, die genetische Determinante gemäß einem der Ansprüche 1 bis 8 aufweist.

10. Nachkommenschaft einer Gurkenpflanze nach einem der Ansprüche 1 bis 8 oder von Gurkensamen nach Anspruch 9, welche die gemäß einem der Ansprüche 1 bis 8 definierte genetische Determinante aufweist.

11. Fortpflanzungsmaterial, das zur Züchtung einer Pflanze nach einem der Ansprüche 1 bis 8 geeignet ist, wobei das Fortpflanzungsmaterial zur geschlechtlichen Fortpflanzung geeignet ist, und insbesondere ausgewählt ist aus der Gruppe, bestehend aus Mikrosporen, Pollen, Eierstöcken, Samenständen, Embryosäcken und Eizellen, oder zur vegetativen Fortpflanzung geeignet ist, und insbesondere ausgewählt ist aus der Gruppe, bestehend aus Stecklingen, Wurzeln, Stängeln, Zellen, Protoplasten, oder für Gewebekulturen regenerierbarer Zellen geeignet ist, und insbesondere ausgewählt ist aus der Gruppe, bestehend aus Blättern, Pollen, Embryonen, Keimblättern, Keimstängeln, meristematischen Zellen, Wurzeln, Wurzelspitzen, Staubbeuteln, Blüten, Samen und Stängeln, wobei die aus dem Fortpflanzungsmaterial gezogene Pflanze die genetische Determinante gemäß einem der Ansprüche 1 bis 8 aufweist.

12. Verwendung eines molekularen Markers, definiert als E13/M51-154.68 oder E16/M60-086.74, zur Identifizierung einer Gurkenpflanze, die im Keimlingsstadium einen dunkleren grünen Stängel aufweist, der im Vergleich zu einer isogenen, die genannte genetische Determinante nicht tragenden Gurkenpflanze auf eine dunklere grüne Farbe der Früchte im Erntestadium schließen lässt.

13. Molekularer Marker zur Identifizierung der gemäß einem der Ansprüche 1 bis 7 definierten genetischen Determinante, wobei der molekulare Marker als E13/M51-154.68 oder E16/M60-086.74 definiert ist.

14. Ein Verfahren zur Züchtung einer Gurkenpflanze, die einen dunkleren grünen Stängel, wie in einem der Ansprüche 1 bis 8 definiert, aufweist, durch Verwendung einer Technik zur Erzeugung von Doppelhaploiden, um eine doppelhaploide Linie zu erzeugen, die homozygot die gemäß einem der Ansprüche 1 bis 8 definierte und zu dem dunkleren grünen Stängel führende genetische Determinante aufweist.

15. Ein Verfahren zur Züchtung einer Gurkenpflanze mit einem dunkleren grünen Stängel, der auf eine dunklere grüne Fruchtfarbe schließen lässt, unter Verwendung von für die vegetative Fortpflanzung oder für Gewebekulturen regenerierbarer Zellen geeignetem, wie in Anspruch 11 definiertem Fortpflanzungsmaterial.

16. Ein Verfahren zur Züchtung einer die gemäß einem der Ansprüche 1 bis 8 definierte genetische Determinante aufweisende Gurkenpflanze durch Ziehen einer Gurkenpflanze aus einem Samen, welcher in seinem Genom die gemäß einem der Ansprüche 1 bis 8 definierte genetische Determinante aufweist.

17. Verfahren nach Anspruch 16, wobei der Samen bei der NCIMB unter der Hinterlegungsnummer NCIMB 41859 hinterlegt ist.

## Revendications

1. Plant de concombre de l'espèce *Cucumis sativus* portant un déterminant génétique qui conduit à une tige d'un vert plus sombre au stade plantule qui est prédictive d'une couleur vert plus sombre des fruits par rapport à un plant de concombre isogénique ne portant pas ledit déterminant génétique, dans lequel ledit déterminant génétique est génétiquement lié aux marqueurs moléculaires E13/M51-154.68 et/ou E16/M60-086.74, et dans lequel la teneur en chlorophylle dans la tige du plant au stade plantule est au moins de 40 µg/g plus élevée ou au moins de 30 % plus élevée que la teneur en chlorophylle dans la tige d'un plant de concombre isogénique au stade plantule ne portant pas ledit déterminant génétique, qui est cultivé dans les mêmes conditions, dans lequel le déterminant génétique est tel qu'il est compris dans un plant de concombre dont une graine représentative a été déposée auprès du NCIMB sous le numéro de dépôt NCIMB 41859.

2. Plant de concombre selon la revendication 1, dans lequel le déterminant génétique est introgressé à partir d'un plant de concombre dont une graine représentative a été déposée auprès du NCIMB sous le numéro de dépôt NCIMB 41859.

3. Plant de concombre selon la revendication 1 ou 2, pouvant être obtenu par croisement d'un premier plant de concombre avec un second plant de concombre, dans lequel au moins l'un desdits plants est cultivé à partir d'une graine dont un échantillon représentatif a été déposé sous le numéro de dépôt NCIMB 41859, ou d'une descendance de celui-ci, facultativement par autofécondation du F1 résultant, et sélection de plants qui présentent une ou plusieurs des caractéristiques suivantes :
a) ils présentent au stade plantule une tige d'un vert plus sombre par rapport à la tige d'une plantule d'un plant de concombre isogénique ne portant pas ledit déterminant génétique, laquelle tige d'un vert plus sombre est prédictive de la production de fruits ayant une couleur vert plus sombre au stade de la récolte par rapport aux fruits d'un plant de concombre isogénique ne portant pas ledit déterminant génétique dans les mêmes conditions, et/ou
b) ils présentent au stade plantule une augmentation de la teneur en chlorophylle dans la tige par rapport à la tige d'une plantule d'un plant de concombre isogénique ne portant pas ledit déterminant génétique, laquelle augmentation de la teneur en chlorophylle est prédictive de la production de fruits ayant une couleur verte plus sombre au stade de la récolte par rapport aux fruits d'un plant de concombre isogénique ne portant pas ledit déterminant génétique dans les mêmes conditions.

4. Plant de concombre selon l'une quelconque des revendications 1 à 3, dans lequel la teneur en chlorophylle dans la tige du plant au stade plantule est au moins, par ordre de préférence croissante, de 40 µg/g, 70 µg/g, 100 µg/g, 120 µg/g, 150 µg/g, 180 µg/g, 200 µg/g plus élevée que la teneur en chlorophylle dans la tige d'un plant de concombre isogénique au stade plantule ne portant pas ledit déterminant génétique, qui est cultivé dans les mêmes conditions.

5. Plant de concombre selon l'une quelconque des revendications 1 à 3, dans lequel la teneur en chlorophylle dans la tige du plant au stade plantule est au moins, par ordre de préférence croissante, de 30 % plus élevée, 60 % plus élevée, 90 % plus élevée, 120 % plus élevée, 150 % plus élevée, 180 % plus élevée, 200 % plus élevée, 230 % plus élevée, 270 % plus élevée, 300 % plus élevée que la teneur en chlorophylle dans la tige d'un plant de concombre isogénique au stade plantule ne portant pas ledit déterminant génétique, qui est cultivé dans les mêmes conditions.

6. Plant de concombre selon l'une quelconque des revendications 1 à 5, dans lequel la couleur verte du fruit depuis le stade de la récolte jusqu'à au moins 14 jours après le stockage, de préférence jusqu'à 21 jours après le stockage, est d'au moins 2 niveaux plus sombre, de préférence d'au moins 3 niveaux plus sombre, sur une échelle de 1 à 9, que la couleur du fruit d'un plant de concombre isogénique ne portant pas ledit déterminant génétique, qui est cultivé et stocké dans les mêmes conditions.

7. Plant de concombre selon l'une quelconque des revendications 1 à 6, dans lequel la couleur verte plus sombre du fruit est en rapport avec une augmentation de la teneur en chlorophylle de la peau du fruit d'au moins, par ordre de préférence croissante, 100 µg/g, 130 µg/g, 160 µg/g, 200 µg/g, 240 µg/g par rapport au fruit d'un plant de concombre isogénique ne portant pas ledit déterminant génétique, qui est cultivé dans les mêmes conditions.

8. Plante de concombre selon l'une quelconque des revendications 1 à 7, dans lequel la couleur verte plus sombre du fruit au stade de la récolte est en rapport avec une augmentation de la teneur en chlorophylle de la peau du fruit d'au moins, par ordre de préférence croissante, 30 %, 40 %, 50 %, 60 %, 70 % par rapport au fruit d'un plant de concombre isogénique ne portant pas ledit déterminant génétique, qui est cultivé dans les mêmes conditions.

9. Graine de concombre, dans laquelle le plant qui peut être cultivé à partir de la graine comprend le déterminant génétique tel que défini dans l'une quelconque des revendications 1 à 8.

10. Descendance d'un plant de concombre selon l'une quelconque des revendications 1 à 8 ou d'une graine de concombre selon la revendication 9, comprenant le déterminant génétique tel que défini dans l'une quelconque des revendications 1 à 8.

11. Matériel de propagation approprié pour produire un plant selon l'une quelconque des revendications 1 à 8, dans lequel le matériel de propagation est approprié pour une reproduction sexuée, et est en particulier sélectionné parmi les microspores, le pollen, les ovaires, les ovules, les sacs embryonnaires et les cellules oeufs, ou est approprié pour une reproduction végétative, et est en particulier sélectionné parmi les boutures, les racines, les tiges, les cellules, les protoplastes, ou est approprié pour les cultures tissulaires de cellules régénérables, et est en particulier sélectionné parmi les feuilles, le pollen, les embryons, les cotylédons, les hypocotyles, les cellules méristématiques, les racines, les pointes de racine, les anthères, les fleurs, les graines et les tiges, dans lequel le plant produit à partir du matériel de propagation comprend le déterminant génétique tel que défini dans l'une quelconque des revendications 1 à 8.

12. Utilisation d'un marqueur moléculaire défini par E13/M51-154.68, ou E16/M60-086.74 pour l'identification d'un plant de concombre qui présente une tige d'un vert plus sombre au stade plantule qui est prédictive d'une couleur verte plus sombre des fruits au stade de la récolte par rapport à un plant de concombre isogénique ne portant pas ledit déterminant génétique.

13. Marqueur moléculaire pour l'identification du déterminant génétique tel que défini dans l'une quelconque des revendications 1 à 7, lequel marqueur moléculaire est défini par E13/M51-154.68, ou E16/M60-086.74.

14. Procédé de production d'un plant de concombre comprenant une tige d'un vert plus sombre tel que défini dans l'une quelconque des revendications 1 à 8, en utilisant une technique de génération de dihaploïde pour générer une lignée de dihaploïde qui comprend de manière homozygote le déterminant génétique tel que défini dans l'une quelconque des revendications 1 à 8 qui donne la tige d'un vert plus sombre.

15. Procédé de production d'un plant de concombre comprenant une tige d'un vert plus sombre qui est prédictive d'une couleur de fruit vert plus sombre, en utilisant un matériel de propagation approprié pour une reproduction végétative ou approprié pour les cultures tissulaires de cellules régénérables tel que défini dans la revendication 11.

16. Procédé de production d'un plant de concombre comprenant le déterminant génétique tel que défini dans l'une quelconque des revendications 1 à 8, en cultivant un plant de concombre à partir d'une graine qui comprend le déterminant génétique tel que défini dans l'une quelconque des revendications 1 à 8 dans son génome.

17. Procédé selon la revendication 16, dans lequel la graine est une graine déposée auprès du NCIMB sous le numéro de dépôt NCIMB 41859.
